**Europäisches Patentamt**

**European Patent Office**

**Office européen des brevets**

(11) Publication number : **0 478 506 A2**

# EUROPEAN PATENT APPLICATION

(21) Application number : **91810754.1**

(22) Date of filing : **26.09.91**

(51) Int. Cl.⁵ : **A61N 5/06**

(30) Priority : **28.09.90 CH 3120/90**

(43) Date of publication of application :
**01.04.92 Bulletin 92/14**

(84) Designated Contracting States :
**AT BE DE DK ES FR GB GR IT LU NL SE**

(71) Applicant : **TECLAS TECNOLOGIE LASER SA**
**Zona Industriale**
**CH-6594 Contone (CH)**

(72) Inventor : **Anelli, Italo, Prof. Dr.**
**V. Ponte Tresa, 7**
**CH-6924 Sorengo (CH)**

(74) Representative : **Moretti, Francesco, Dr.**
**e/o ENGIMPEX S.A., Industrial Property Serv.**
**Dept., Via Ponte Tresa 7b, P.O. Box 12**
**CH-6924 Sorengo (CH)**

(54) **Dedicated red-radiating equipment for photochemotherapy.**

(57)    The equipment includes a hot-light lamp with good emission on the red and low IR and UV radiation, suitably filtered to obtain radiating energy in the required frequency band.

The lamp is associated with a drug photosensitive to the red radiation emitted by it and finds use in the treatment of the psoriasis.

Unlike the methods and means of the known technique such equipment does not show risks or collateral effects of considerable importance both because of the harmlessness of the emitted radiation and the bearableness of the associated drug.

Moreover with the equipment, itself cheaper than the known ones, we can reduce drastically both the number of sessions, which can be performed at home too and not under medical control in hospital or surgery, and the associated medical and social costs.

EP 0 478 506 A2

The present invention refers to a dedicated red-radiating equipment for photochemotherapy, in particular for the treatment of the psoriasis by means of the use of a hot-light source associated with photosensitive drugs.

The present invention allows a therapy much less dangerous than those already known both from the point of the radiation, pratically harmless visible light unlike the carcinogenic UV-radiation, and from the point of view of the limited use of much more tolerable drugs without serious collateral effects, being these drugs activated by said kind of radiation.

For the treatment of the psoriasis it is currently used a photochemotherapeutic methodology, so called PUVA. This universally adopted system consists in administering a drug, called PSORALENE, (8-MOP), psoralenic acid, 8-metoxypsorolen, activated by the UV-radiation in external topical application, ointments, or by means of preventive ingestion, tablets, and in irradiating the concerned parts with UV-light, branch A, subsequently.

A recession of the psoriatic manifestation is obtained with about 50 half-hour sessions four times a week on an average. The patient shows a relapse always on an average after 1, 2 or 3 months and then undergoes the therapeutic cycle again.

It must be said that the psoriasis is considered a social disease in many countries (in Italy the 3% of the population is subject to more or less serious and diffuse forms of this disease). It cannot be considered evidently the best a cycle of treatment which foresees a continuous assumption of drugs and especially a so extended exposition to the notoriously carcinogenic UV-rays, even if represents the only universally adopted solution.

It must be added that the drug has well-known toxic and mutagenic characteristics. Moreover the treatment must be done in clinic or hospital under control, involving a high social cost both by the loss of hours/work and by the charge to the sickness funds. The basic concept of a photochemotherapeutic treatment is to administer a "photosensitive" drug, which is activated namely by the light at a determined wavelength characteristic of the drug.

The PUVA system is just so called by:

P = psoralene
UV = ultra-violet
A = branch A

The invention of the applicant allows now to reach a very distinct and radical reduction of the sessions instead, by means of the use of an absolutely harmless radiating emission and much more harmless drugs sensitive to this radiation.

The subject of the present invention consists in using red-light-emitting lamps coupled with drugs photosensitive just to the light having this wavelength.

As a result the recession is tipically obtained after just a half-hour session, at the most two. It follows that the drug is administered to the patient just few times a year and on those few occasions he is irradiated this happens with a totally harmless red light.

Because it is necessary to irradiate the most different surfaces of the human body (small like elbows, knies and scalp, middle like back and legs or the total body) the photoemitting systems can have different dimensions, but with equivalent technological criteria.

The basic concept is anyway to use lamps having a low emission both in ultra-violet and infra-red like for instance the fluorescent lamps associated with optical screens which let the light through only in the required frequency band. The present invention refers more precisely to a dedicated equipment for photochemotherapy in particular for the treatment of the psoriasis, characterized in that it includes a hot-light fluorescent lamp or photo-emitter, means for the determination of the selected range of wavvlengths, red light, able to activate drugs photosensitive to said radiation.

To obtain a better efficency lamps are selected with a heat temperature less or equal to 3000 °K, in order both to minimize the dangerous UV radiation at the source and at the same time to minimize the cooling problems, avoiding an excessive heating both of the system itself and of the skin of the patient.

Finally an optical filter is placed in front of the lamps, said filter allowing only the light through at the desidered wavelength, typically between 600 and 700 nanometer, i.e. red light.

The use of the so called "hot-light" fluorescent tubes is to be preferred because:

– they can have different dimensions (small, middle, large) as previously indicated;
– they have a low IR emission;
– in the hot-light version they have a low UV emission;
– such low UV and IR emissions can be easily filtered by the optical filter placed in front of the lamps.

Finally such lamps are provided with timers which allow to preselect the time necessary to irradiate the required amount of energy of light, equal to some tens of Joule/cm$^2$, in function of the photosensitive drug and the therapy to be applied to the patient.

As associated photosensitive drug it is used the ptalocyanine activated by light with frequency range between 600 and 700 nanometer, harmless red, and which shows good bearableness or other derivatives or equivalents.

It is interesting to note, and this is the high merit of the present invention, that the foreseen lamps are those with a good red emission and low UV and IR emission and then cheaper than the UVA lamps. In particular the so called hot-light fluorescent tubes can be used.

We give in the following a short, anyway not limiting, description of the subject of the present invention, without taking anything away from the generality of the invention itself.

The equipment of the invention is composed of a fluorescent lamp suitably doped on the red by means of screens or filters, coloured glasses, in order to obtain the required radiation, red light, frequency band between 600 and 700 nanometer, to avoid the excessive heating of the skin (IR radiation) and to eliminate the dangerous UV radiation (carcinogenic).

It is of dimension suitable to the part of the surface of the body to be treated and of ergonomic shape suitable to the shape of the part of the body undergoing the therapy.

It is foreseen also its use in form of modular elements.

We can conclude that the novelty of the present invention consists in associating low-cost hot-light lamps with drugs pphotosensitive to said emissions, utilizing existing UV-emitting systems, suitably adapted, to obtain a bearable treatment without risks with drastic reduction of the duration of the sessions, checks and costs.

## Claims

1) Dedicated equipment for photochemotherapy, in particular for the treatment of the psoriasis, characterized in that it includes a hot-light fluorescent lamp or photo-emitter, means for the determination of the selected range of wavelengths, red light, able to activate drugs photosensitive to said radiation.

2) Dedicated equipment for photochemotherapy according to the claim 1, where the lamp is a fluorescent lamp of the hot-light type, typically with high emission in wavelength band between 600 and 700 nanometer, red light, and low UV and IR emission.

3) Dedicated equipment for photochemotherapy according to the claim 1 or 2, when the means of definition of the frequency band of the radiation are optical filters.

4) Dedicated equipment for photochemotherapy according to any one of the claims 1 to 3, associated to accessories like interchangeable filters and/or devices of delivery of the light and peculiar drugs photosensitive to said radiation.

5) Dedicated equipment for photochemotherapy according to the claim 4, where the drug photosensitive to the red emission is the ptalocyanine or equivalent.

6) Dedicated equipment for photochemotherapy according to any one of the claims 1 to 5, characterized in that it includes timers of the duration of the emission programmable according to the therapy imposed to the patient and to the type of associated drug.

7) Dedicated equipment for photochemotherapy according to any one of the claims 1 to 6, characterized in that it shows dimensions and ergonomic shapes suitable to the surfaces of the human body to be irradiated, being these small or middle or the total body.

8) Dedicated equipment for photochemotherapy according to any one of the claims 1 to 7, characterized in that it is modular.